# EUROPEAN PATENT APPLICATION

(11) **EP 2 206 460 A1**
(43) Date of publication of application: **14.07.2010**
(21) Application number: 09004845.5
(22) Date of filing: 01.04.2009
(51) Int. Cl.: A61B 1/227, A61B 1/24, A61B 3/13, A61B 5/103, G02B 21/36, A61B 1/247, A61B 3/14

(54) **Optical inspection apparatus with a detachable light guide**

(30) Priority: 07.01.2009 US 349731
(71) Applicant: AnMo Electronics Corporation,, Hsinchu City (TW)
(72) Inventor: Wu, Paul Neng-wel, Hsinchu City (TW)
(74) Representative: Zeller, Andreas

(57) **Abstract**

An optical inspection apparatus is disclosed including: a digital image sensing module, a light source, a detachable light guide configured for guiding incident light to transmit within the detachable light guide and to output from a first angle, and an adapter engaged with the digital image sensing module for guiding light emitted from the light source to the detachable light guide; wherein the detachable light guide is detachably engaged with the adapter.

## Description

### BACKGROUND OF THE INVENTION

The present invention relates to an optical inspection apparatus according to the precharacterizing clause of claim 1 or claim 2, and a light guide device according to the precharacterizing clause of claim 12.

Different types of digital image sensing systems are developed for different applications. It is easy to observe that the image obtaining and signal processing are the same for different types of digital image sensing systems in different applications according to the related art. Moreover, these different types of digital image sensing systems are equipped with the same electronic cameras. Only the objective head portions are different for accommodating different applications.

This in mind, the present invention aims at providing an optical inspection apparatus that can share the identical (or substantially similar) core digital image sensing portion.

This is achieved by an optical inspection apparatus according to claim 1 or claim 2, or a light guide device according to claim 12. The dependent claims pertain to corresponding further developments and improvements.

As will be seen more clearly from the detailed description following below, the claimed optical inspection apparatus includes a detachable light guide detachably engaged with an adapter.

In the following, the invention is further illustrated by way of example, taking reference to the accompanying drawings. Thereof
FIG. 1 is a schematic diagram of a digital image sensing system according to the related art,
FIG. 2 is a schematic diagram of an intra-oral inspection system according to the related art,
FIG. 3 is a schematic diagram of an optical inspection apparatus with an ophthalmology-compatible light guide according to an exemplary embodiment,
FIG. 4 is an exploded view of the optical inspection apparatus with the ophthalmology-compatible light guide of FIG. 3,
FIG. 5 is a sectional view of the optical inspection apparatus with the ophthalmology-compatible light guide of FIG. 3,
FIG. 6 is a schematic diagram of an optical inspection apparatus with a dermatology-compatible light guide according to an exemplary embodiment,
FIG. 7 is an exploded view of the optical inspection apparatus with the dermatology-compatible light guide of FIG. 6,
FIG. 8 is an exploded diagram of an optical inspection apparatus with a probing light guide according to an exemplary embodiment,
FIG. 9 is an exploded diagram of an intra-auricular compatible optical inspection apparatus according to an exemplary embodiment,
FIG. 10 is a sectional view of the intra-auricular compatible optical inspection apparatus of FIG. 9,
FIG. 11 is a schematic diagram of a reflecting-type optical inspection apparatus according to an exemplary embodiment, and
FIG. 12 is an exploded view of the reflecting-type optical inspection apparatus of FIG. 11.

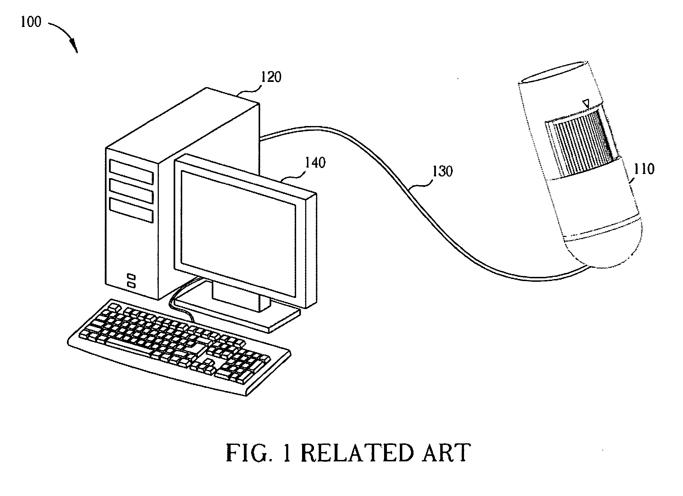

FIG. 1 shows a digital image sensing system 100 according to the related art. The digital image sensing system 100 includes an electronic camera 110, a computer 120 connected to the electronic camera 110 via a cable 130, and a display 140. The electronic camera 110 may be used to obtain an image of an object. The image signal would be transmitted via the cable 130 to the computer 120 and displayed on the display 140, and the image signal may be saved in or further processed by the computer 120.

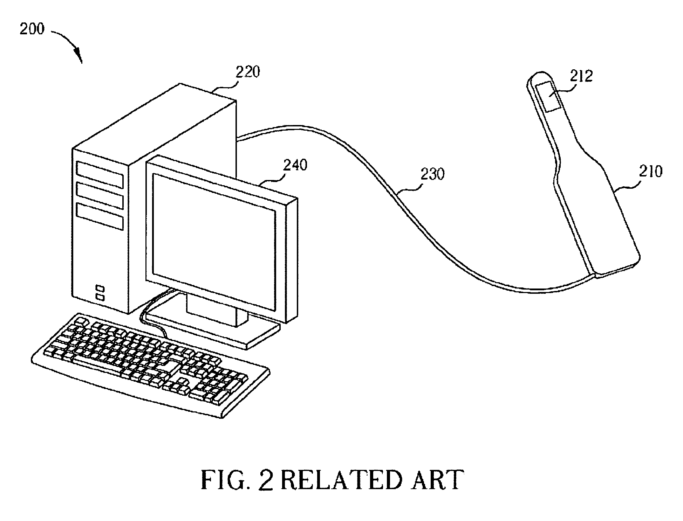

Different types of digital image sensing systems are developed for different applications. For example, intra-oral inspection systems are widely adopted for dental or oral inspection. FIG. 2 shows an intra-oral optical inspection system 200 according to the related art. The intra-oral inspection system 200 includes an intra-oral camera 210, a computer 220 connected to the intra-oral camera 210 via a cable 230, and a display 240. As shown in FIG. 2, the conventional intra-oral optical inspection system 200 has an elongate shape with a camera module 212 situated in the head portion. To observe oral tissue, a diagnostician would insert the head portion and part of the elongate handle of the intra-oral camera 210 into a patient's mouth cavity. Similarly, the image signal would be transmitted via the cable 230 to the computer 220 and displayed on the display 240.

Intra-auricular optical inspection systems are another example. The intra-auricular optical inspection system may need to have an even thinner and curved shaped handle, covered with an elastic sheath in the head portion for protecting the ear drum of the patient under inspection. However, the image obtaining and signal processing are still similar to the digital image sensing system 100 shown in FIG. 1 and the conventional intra-oral optical inspection system 200 shown in FIG. 2.

In the following description of the present invention, some embodiments may be described in the context of dentistry, ophthalmology, otology, dermatology, and packaging industry; however, such descriptions are exemplary only and shall in no way be interpreted to limit the use of the present invention to the fields above. The optical inspection apparatus with a detachable light guide and related system of the present invention have practical applications in many fields including, but not limited to, automotive applications, medicine-related applications, industrial applications, and personal hobby applications.

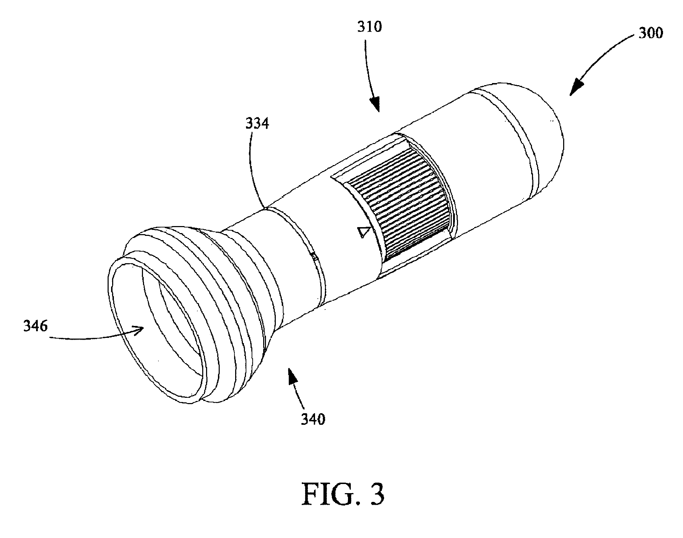

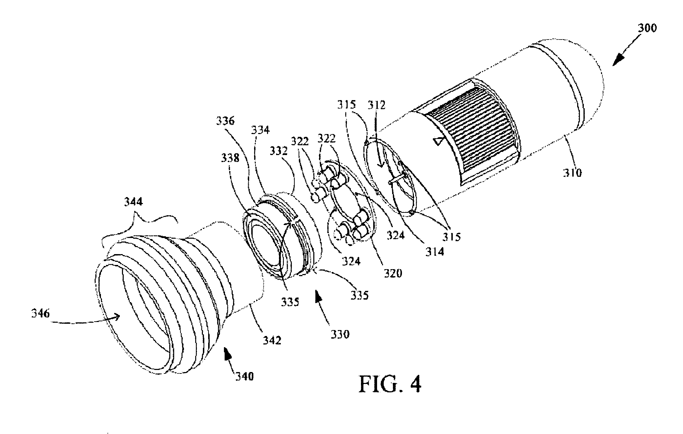

FIG. 3 illustrates a schematic diagram of an optical inspection apparatus 300 with an ophthalmology light guide 340, according to an exemplary embodiment. The optical inspection apparatus 300 comprises a digital image sensing module 310, a light source 320 (not shown in FIG. 3), an adapter 330 with only an annular part 334 shown in FIG. 3, and an ophthalmology light guide 340 with an opening 346. FIG. 4 is an exploded view of the optical inspection apparatus 300 of FIG. 3. As shown in FIG. 4, the light source 320 may include, for example, a plurality of LEDs 322, for illuminating and enhancing the image sensing of the digital image sensing module 310. The digital image sensing module 310 may include a fastener 314 for coupling with a jack 324 of the light source 320 to fasten the light source 320. The digital image sensing module 310 may further include a plurality of protrusions 315 to wedge with cavities 335 on the annular portion 334 of the adapter 330 according to the exemplary embodiment. The adapter 330 is configured to receive the light emitted from the light source 320. The adapter 330 according to the exemplary embodiment is made of a kind of photo conductor such that the incident light may transmit along a predetermined path within the adapter 330 and output at an output end 338 to the detachable light guide 340. The ophthalmology-compatible light guide 340 may be detachably engaged with the adapter 330. According to the exemplary embodiment, the ophthalmology-compatible light guide 340 is configured for guiding incident light to transmit along a predetermined path within the ophthalmology-compatible light guide 340 and to output from the inner plane 344. The detachable light guide 340 may be manufactured from a type of photo conductor, which includes but is not limited to transparent plastic.

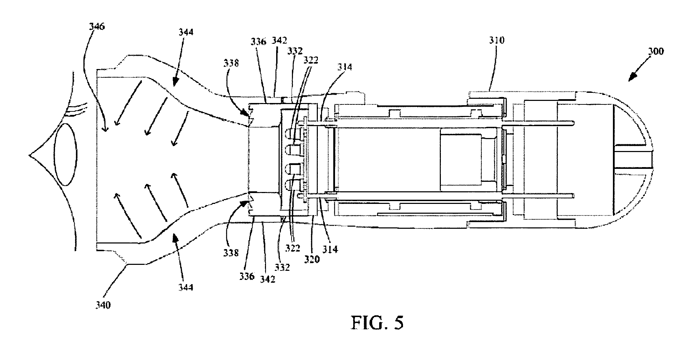

FIG. 5 is a sectional view of the present optical inspection apparatus 300 of FIG. 3. When fabricating the optical inspection apparatus 300 of the present invention, the light source 320 is disposed at an objective end of the digital image sensing module 310 and engaged with the digital image sensing module 310. Referring to FIG. 4 and FIG. 5, the light source 320 is disposed within the hollow portion 312 of the digital image sensing module 310 and fastened with the digital image sensing module 310 by the fasteners 314 of the digital image sensing module 310. An upper portion 332 of the adapter 330 is disposed at the objective end of the digital image sensing module 310, stretching into the hollow portion 314 of the digital image sensing module 310. The ophthalmology-compatible light guide 340, accommodating the lower portion 336 of the adapter 330, is detachably engaged with the adapter 330. As illustrated in FIG. 5, the LEDs 322 of the light source 320 are directed to the adapter 330. The light output from the output end 338 of the adapter 330 is then received by the ophthalmology-compatible light guide 340 and output from the inner plane 344. As shown in FIG. 5, the arrows pointing out from the inner plane 344 represent the directions of the light output from the ophthalmology-compatible light guide 340. A diagnostician could inspect a patient's eye, for example, an iris inspection, by aiming the opening 346 of the ophthalmology-compatible light guide 340 of the optical inspection apparatus 300 at the patient's eye. The light emitted from the LEDs 322 of the light source 320 is guided by the adapter 330 and the ophthalmology-compatible light guide 340 and outputted from the inner plane 344 of the ophthalmology light guide 340.

According to this exemplary embodiment, the shape of plane 344 is similar to a cone section. Hence, the incident light, outputted from the inner plane 344, enters the patient's eye at an acute angle. Therefore, light reflection is reduced and the optical inspection and image sensing of the eye or tissue by the digital image sensing module 310 may be enhanced. Please be noted that the optical inspection apparatus 300 may be utilized to perform inspection of other objects and provide illumination with reduced light reflection as well.

The present invention provides an optical inspection apparatus with a detachable light guide to accommodate different applications. Different light guides are configured for different applications and may be chosen according to the application to detachably engage with the digital image sensing module or the adapter for guiding the light to a target object under inspection for enhancing the illumination in the inspection. In operation, an user could choose a detachable light guide according to the desired inspection and attach the chosen detachable light guide to a digital image sensing module. The light emitted by the light source coupled with the objective end of the digital image sensing module is then inputted to the adapter and then enters the detachable light guide. The incident light to the detachable light guide would transmit within the detachable light guide and then output from a desired angle to the target object.

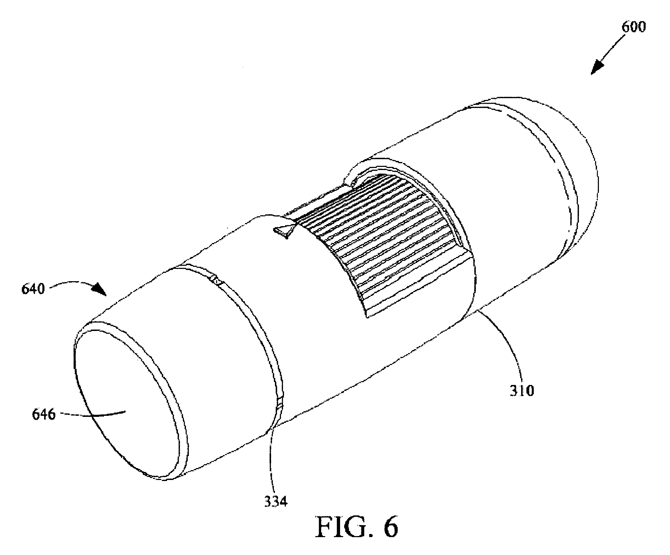

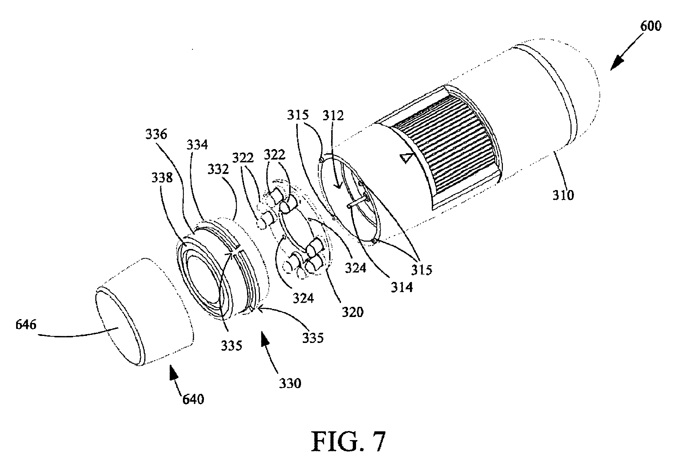

FIG. 6 is a schematic diagram of an optical inspection apparatus 600 with a dermatology-compatible light guide, according to another exemplary embodiment. The optical inspection apparatus 600 of the present invention comprises a digital image sensing module 310, a light source 320 (not shown in FIG. 6), an adapter 330 with only a part 334 shown in FIG. 6, and a detachable dermatology-compatible light guide 640 with a pressing sheet 646. FIG. 7 is an exploded view of the optical inspection apparatus 600 of FIG. 6. The structure of the digital image sensing module 310, the light source 320 and the adapter 330 are the same as the corresponding components of the optical inspection apparatus 300 shown in FIG. 3, FIG. 4 and FIG. 5. The pressing sheet 646 of the detachable dermatology-compatible light guide 640 is configured for pressing an area of skin under inspection to improve the dermatology inspection. The optical inspection apparatus 600 of the present invention with the detachable light guide 640 may have inspection applications in other fields. The pressing sheet 646 may be utilized to press against other objects of interest, and need not be limited to skin inspection. The pressing sheet 646 and the dermatology-compatible light guide 640 may be formed integrally or separately.

The exemplary embodiments shown in FIG. 3 to FIG. 7 illustrate how the present invention reduce cost by utilizing shareable components. E.g., depending on the intended optical inspection, a suitable detachable light guide can be selected to work with compatible digital image sensing module 310, light source 320, and adapter 330. By utilizing different light guides, the same digital image sensing module may be applied to different fields.

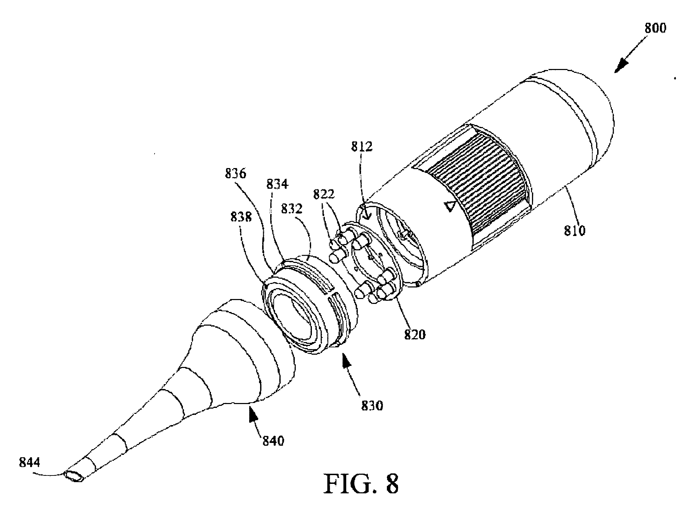

The present invention further provides an optical inspection apparatus with a detachable light guiding portion, which includes an adapter and a light guide, to accommodate different applications. Please refer now to FIG. 8, which is an exploded diagram of an optical inspection apparatus 800 with a probing light guide 840 according to an exemplary embodiment. As shown in FIG. 8, the optical inspection apparatus 800 of the present invention comprises a digital image sensing module 810, a light source 820 disposed within a hollow portion 812 at an objective end of the digital image sensing module 810, and a detachable light guiding portion including an adapter 830 and a probing light guide 840 with an output end 844. The probing light guide 840 comprises an elongate shape, capable of guiding incident light to transmit within the light guide 840 and output at the output end 844 to enhance the illumination on an object to be inspected. In this embodiment, the structure of the digital image sensing module 810 and the light source 820 are similar to the corresponding components of the optical inspection apparatuses 300 and 600. However, the adapter 830 according to this exemplary embodiment is detachably engaged with the digital image sensing module 810. The detachable adapter 830 provides flexibility in adaptation to different light guides. For example, the probing light guide 840 comprises an elongate shape for provide better probing into narrow openings or crevices. If, in a different example, an optical inspection apparatus 800 is designed to be compatible with adapter 330 (same as the one used for optical inspection apparatuses 300 and 600), then, to facilitate proper integration, the probing light guide 840 may need to be as wide as the adapter 330 at one end. But in order to guide the light to output end 844 with the same efficiency, the probing light guide 840's shape will be characterized by a wide integration end (for integrating with optical inspection apparatus) that rapidly shrinks into a narrow objective head at another end. This shape increases the technical difficulty for designing the light guide. In FIG. 8, the optical inspection apparatus 800 utilizes a detachable adapter 830, which may be well designed to match one specific light guide for the desired application.

The optical inspection apparatus 800 with the probing light guide 840, as shown in FIG. 8, is applicable to various electronics-related areas, such asprinted circuit board (PCB) inspections. For instance, bonding reliability is of high importance in the PCB industry. The detachable probing light guide of the present invention could be utilized to help check the bonding of chips and other components on the PCB. Aside from the above specific example, the optical inspection apparatus 800 can also be utilized in other electronics-related applications where the visual target of interest is otherwise difficult to view.

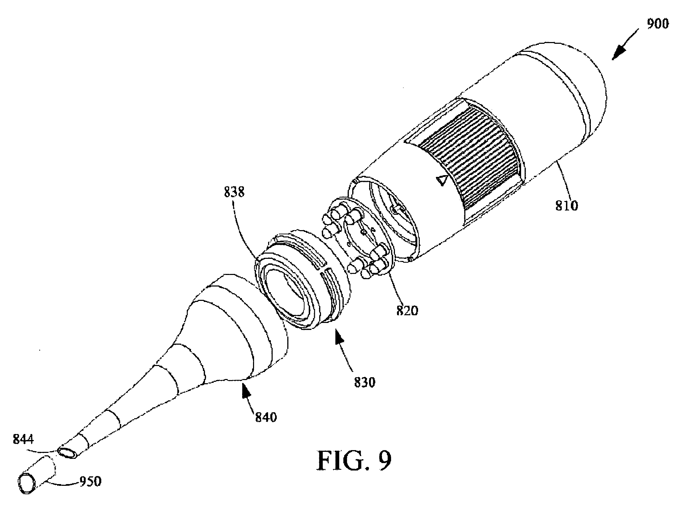

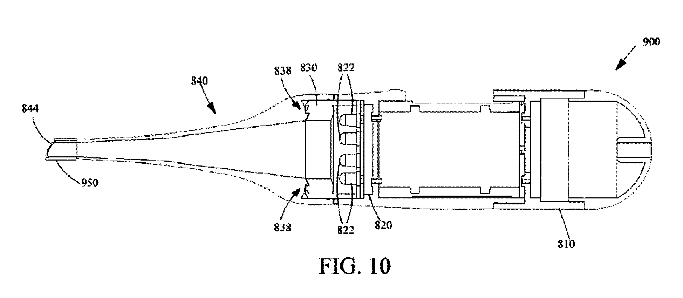

FIG. 9 is an exploded diagram of an intra-auricular compatible optical inspection apparatus 900 according to an exemplary embodiment, and FIG. 10 is a sectional view of the intra-auricular compatible optical inspection apparatus of FIG. 9. As shown in FIG. 9 and FIG. 10, the intra-auricular compatible optical inspection apparatus 900 comprises components similar to the optical inspection apparatus 800 with an ear sheath 950 covering the probing light guide 840. The light source 820 is engaged with the digital image sensing module 810, the detachable adapter 830 is detachable engaged with the digital image sensing module 810, and the light guide 840 is engaged with the detachable adapter 830 or with the digital image sensing module 810. In operation, the light emitted from the LEDs 822 is inputted to the detachable adapter 830 and outputted at the output end 838, and then inputted to the light guide 840 and transmitted. The light is then outputted at the output end 844 to enhance the illumination for the intra-auricular inspection. The ear sheath 950 may be made of soft and elastic material for protecting the ear drum of the patient under inspection. A diagnostician may put the head portion of the intra-auricular compatible optical inspection apparatus 900, covered with the ear sheath 950, into the ear of the patient under inspection.

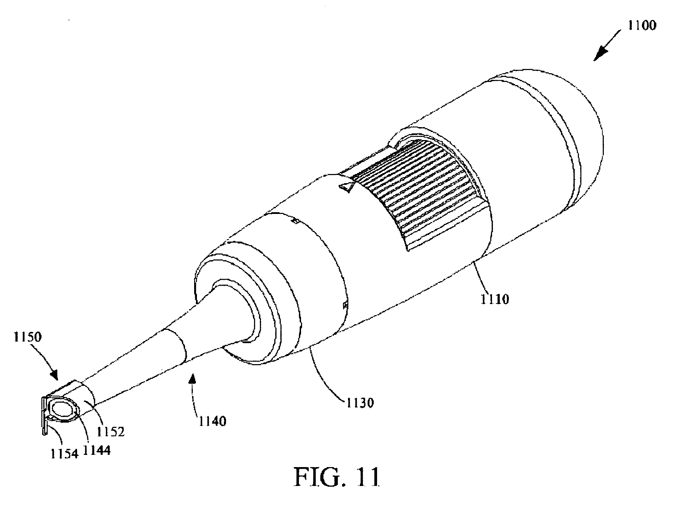

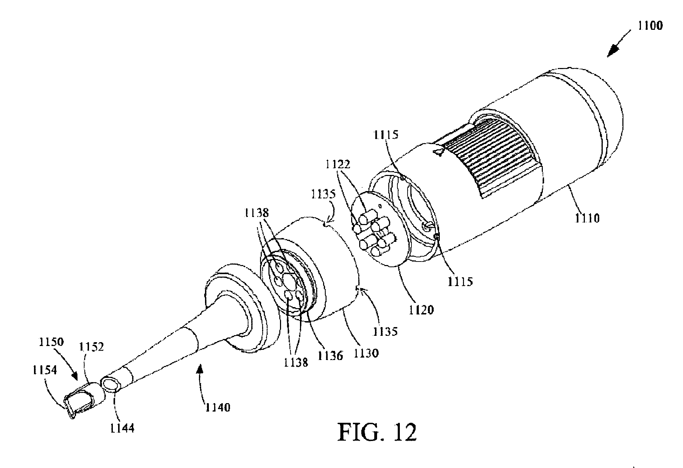

FIG. 11 is a schematic diagram of a reflecting-type optical inspection apparatus 1100 according to an exemplary embodiment, and FIG. 12 is an exploded view of the reflecting-type optical inspection apparatus 1100 of FIG. 11. The reflective-type optical inspection apparatus 1100 comprises a digital image sensing module 1110, a light source 1120, an adapter 1130, a detachable light guide 1140 and a reflective kit 1150. The light source 1120 may include, for example, a plurality of LEDs 1122. The light source 1120 is disposed directed to the adapter 1130. The adapter 1130 includes a plurality of through holes 1138. The digital image sensing module 1110 may include a plurality of protrusions 1115 to wedge with cavities 1135 of the adapter 1130 according to the exemplary embodiment. The adapter 1130 may be made of a kind of opaque medium such that the incident light of the detachable light guide 1140, which is emitted from the light source 1120 and inputted to the adapter 1130, is guided by the adapter 1130 so that the light passes through the through holes 1138 of the adapter 1130. The incident angle of the light inputted to the detachable light guide 1140 is then guided to a predetermined angle. The detachable light guide 1140 is detachably engaged with a lower portion 1136 of the adapter 1130. The detachable light guide 1140 receives the light transmitted through the through holes 1138, and the incident light is then transmitted within the detachable light guide 1140 along a predetermined path. The light than is outputted at an output end 1144 of the detachable light guide 1140 for enhancing inspection illumination. The reflective kit 1150 includes a tube 1152 and a reflective surface 1154 adjustably connected to the tube 1152. The tube 1152 is capped on the head portion of the detachable light guide 1140 to fasten the reflective surface 1154 with the light guide 1140. The digital image sensing module 1110 senses light reflected from the reflective surface 1154 to obtain images of a target object. The reflective surface 1154 helps to implement the optical inspection to a target object in a narrow opening, crevice, cranny, or a deep corner. In other words, the reflective surface 1154 helps to inspect areas that are otherwise obstructed, e.g., behind a tooth, a target object under or behind another object, such as a pin disposed at the bottom of an integrated chip (IC).

In some embodiments, the detachable light guide of the present invention may be detachably engaged with the digital image sensing module or the adapter. In other embodiments, the optical inspection apparatus may adopt the detachable adapter detachably engaged with the digital image sensing module. The detachable engagements may be achieved by rotating, clamping, screwing, wedging by cooperation of protrusions and cavities, fastening, slidable-engagement, or by any other way of joining. These are simply exemplary embodiments and should not be interpreted to restrict the present invention in any way.

The adapter adopted in the optical inspection apparatus of the present invention is for guiding the light emitted from the light source so that it properly enters into the light guide to accommodate specific applications. As illustrated in the foregoing descriptions, the adapter may be made of photo conductor material so that the light could transmit within the adapter along a predetermined path. The adapter of the present invention may comprise opaque medium with through holes such that the input light can only transmit through the through holes according to other exemplary embodiments. These implementations are merely exemplary embodiments and should not be interpreted to restrict or limit the present invention in any way. Any other design of the adapter that implements the guiding of incident light to the light guide should be included in the scope of the present invention.

In the above descriptions, the optical inspection apparatuses of the present invention could communicatively couple with a computer and transfer the sensed image signals to the computer for processing, and deliver the image to show on a display. The term "couple" used herein is intended to mean any indirect or direct connection (e.g., a wired or wireless communication connection). The optical inspection apparatuses of the present invention could also be configured to couple with other instruments for further processing. The optical inspection apparatus of the invention could share data, such as image data, with other computers, mobile phones, or other instruments via the internet, local area network (LAN), or other types of communicative networks (e.g., wireless networks). In one example, image captured by an optical inspection apparatus is transmitted to a first device (e.g., a computer, a mobile computing device, a printer, a display, and/or a mobile phone), and then transmitted, via a wired or wireless communicative connection, to one or more devices by the first device. In another example, the image captured by an optical inspection apparatus is simultaneously (or without substantial time difference) shared or transmitted to one or more devices (e.g., computer(s), mobile computing device(s), printer(s), display(s), and/or mobile phone(s)).

## Claims

1. An optical inspection apparatus (300, 600, 1100) comprising:
a digital image sensing module (310, 1110) for sensing light;
a light source (320, 1120) engaged with the digital image sensing module (310, 1110); and
**characterised by**:
a detachable light guide (340, 640, 1140) configured for guiding light input to the detachable light guide (340, 640, 1140) to transmit along a first path within the detachable light guide (340, 640, 1140) and to output from the detachable light guide (340, 640, 1140) from a first angle; and
an adapter (330, 1130) engaged with the digital image sensing module (310, 1110) and the detachable light guide (340, 640, 1140), the adapter (330, 1130) configured for guiding light emitted from the light source (320, 1120) to the detachable light guide (340, 640, 1140) from a second angle, the adapter (330, 1130) being detachably engaged by the detachable light guide (340, 640, 1140).

2. An optical inspection apparatus (800, 900) comprising:
a digital image sensing module (810) for sensing light;
a light source (820) engaged with the digital image sensing module (810); and
**characterised by**:
a detachable light guiding portion comprising:
a detachable adapter (830) detachably engaged with the digital image sensing module (810), the detachable adapter (830) configured for guiding light emitted from the light source (820) to output the detachable adapter (830) from a first angle; and
a light guide (840) engaged with the detachable adapter (830), the light guide (840) configured for guiding light input to the light guide (840) to transmit along a first path within the light guide (840) and to output from the light guide (840) from a second angle.

3. The optical inspection apparatus (300, 600, 800, 900, 1100) of claim 1 or claim 2, **characterised in that** the adapter (330, 830, 1130) is configured for guiding light inputted from the light source (320, 820, 1120) to transmit along a second path within the adapter (330, 830, 1130) and to output to the light guide (340, 640, 840, 1140).

4. The optical inspection apparatus (300, 600, 800, 900, 1100) of claim 1 or claim 2, **characterised in that** the adapter (330, 830, 1130) comprises at least one through hole (1138), wherein an incident angle of the light emitted from the light source (320, 820, 1120) to the light guide (340, 640, 840, 1140) is guided by the adapter (330, 830, 1130) after the light from the light source (320, 820, 1120) transmits through the through hole (1138) of the adapter (330, 830, 1130).

5. The optical inspection apparatus (300, 600, 800, 900, 1100) of claim 1 or claim 2, **characterised in that** the light guide (340, 640, 840, 1140) comprises a tubular portion, in which a radius corresponding to one part of the tubular portion at an end closer to the light source (320, 820, 1120) differs from the radius corresponding to another part of the tubular portion at an end farther from the light source (320, 820, 1120).

6. The optical inspection apparatus (300, 600, 800, 900, 1100) of claim 5, **characterised in that** the light guide (340, 640, 840, 1140) is configured to output light from an inner surface (344) of a section of the tubular portion.

7. The optical inspection apparatus (300, 600, 800, 900, 1100) of claim 1 or claim 2, **characterised in that** an incident angle of the light output from the light guide (340, 640, 840, 1140) to a tangent plane of an object to be inspected is an acute angle.

8. The optical inspection apparatus (300, 600, 800, 900, 1100) of claim 1 or claim 2, **characterised in that** the light guide (340, 640, 840, 1140) comprises a pressing sheet (646) for pressing an area of an object to be inspected.

9. The optical inspection apparatus (300, 600, 800, 900, 1100) of claim 8, **characterised in that** the light guide (340, 640, 840, 1140) and the pressing sheet (646) are integrally formed.

10. The optical inspection apparatus (300, 600, 800, 900, 1100) of claim 1 or claim 2 **further characterised by** a reflective surface (1154) engaged with the light guide (340, 640, 840, 1140), wherein the digital image sensing module (310, 810, 1110) is configured for sensing light reflected from the reflective surface (1154).

11. The optical inspection apparatus (300, 600, 800, 900, 1100) of claim 1 or claim 2, **characterised in that** the light source (320, 820, 1120) comprises a plurality of light emitting diodes (LEDs) (322, 822, 1122), and the plurality of LEDs (322, 822, 1122) are disposed directed at the adapter (330, 830, 1130).

12. A light guide device for a digital optical inspection apparatus, comprising:
an adaptor portion (330, 830, 1130) for joining the light guide device with a digital optical inspection apparatus (300, 600, 800, 900, 1100), wherein the adaptor portion (330, 830, 1130) facilitates directing light from a light source (320, 820, 1120) and outputting the light at a first angle; and
**characterised by**:
a light guide portion (340, 640, 840, 1140) for further directing light from the light source (320, 820, 1120) along a first path within the light guide portion (340, 640, 840, 1140), and wherein the further directed light is emitted at a second angle to illuminate a target object.

13. The light guide device of claim 12, **characterised in that** the adaptor portion (330, 830, 1130) is removably or fixedly engaged with the digital optical inspection apparatus (300, 600, 800, 900, 1100).

14. The light guide device of claim 12, **characterised in that** the adaptor portion (330, 830, 1130) is removably engaged with the light guide portion (340, 640, 840, 1140).

15. The light guide device of claim 12, **characterised in that** the adapter portion (330, 830, 1130) is configured for guiding light from the light source (320, 820, 1120) to transmit along a second path within the adapter portion (330, 830, 1130) and into the light guide portion (340, 640, 830, 1140) at the first angle.

16. The light guide device of claim 12, **characterised in that** the adaptor portion (1130) includes one or more through holes (1138) that light from the light source (1120) can pass through.

17. The light guide device of claim 12, further **characterised by** a generally tubular appearance, and wherein the radius corresponding to one part of the light guide device differs from the radius corresponding to another part of the light guide device.

18. The light guide device of claim 17, further **characterised by** being configured to output light from an inner surface (344) of a section of the tubular portion.

19. The light guide device of claim 12, further **characterised by** a pressing sheet (646) for pressing an area of a target object.

20. The light guide device of claim 19, **characterised in that** the light guide device and the pressing sheet (646) are integrally formed.

21. The light guide device of claim 12, further **characterised by** a reflective light-capturing portion (1154) for sensing light not directly incident upon image sensing modules (1110) of a digital optical inspection apparatus (1100).
